# EUROPEAN PATENT APPLICATION

(11) **EP 4 517 290 A1**
(43) Date of publication of application: **05.03.2025**
(21) Application number: 23796533.0
(22) Date of filing: 28.04.2023
(51) Int. Cl.: G01N 1/04, B01D 43/00, C12M 1/26, C12M 1/34, G01N 33/48

(54) **MICROBE ACCUMULATION METHOD AND ACCUMULATION SYSTEM**

(30) Priority: 28.04.2022 JP 2022074798
(71) Applicant: University Public Corporation Osaka, Osaka City 545-0051 (JP)
(72) Inventor: TOKONAMI, Shiho, Sakai-shi, Osaka 599-8531 (JP); IIDA, Takuya, Sakai-shi, Osaka 599-8531 (JP)
(74) Representative: Bals & Vogel Patentanwälte PartGmbB
(86) International application number: PCT/JP2023/016809
(87) International publication number: WO 2023/210798

(57) **Abstract**

A microorganism accumulation method includes first to third steps to accumulate a plurality of types of microorganisms. The first step is preparing an accumulation substrate (13) provided with a plurality of pores (131). Each of the plurality of pores (131) includes an opening that is capable of capturing at least one microorganism for each of the plurality of types and a depth that extends in a direction including a vertically downward component. The second step is setting a laser light irradiation condition. The third step is irradiating the plurality of pores (131) with laser light through a sample in accordance with the irradiation condition. A region irradiated with the laser light does not include a photothermal conversion material. The setting of the irradiation condition includes setting intensity of non-resonant light within an irradiation range of the non-resonant light such that a magnitude of a vertically downward component of a light-induced force caused by irradiation with the non-resonant light (i) is greater than a magnitude of a vertically upward component of a buoyancy force caused by the liquid sample and (ii) is greater than a magnitude of a vertically upward component of a force caused by a Brownian motion of molecules in the liquid sample.

## Description

### TECHNICAL FIELD

The present disclosure relates to a microorganism accumulation method and an accumulation system, and more specifically to a method and a system to accumulate a plurality of types of microorganisms floating in a liquid sample.

### BACKGROUND ART

In human intestines, oral cavities, skins, and the like, a large number of types of microorganisms are colonized and form microbial flora that is also called "microbiota". The fact that the microbiota is deeply involved in human health (such as diseases and biological reactions) has become increasingly clear such as through analysis of "microbiome" which is the complete set of genome information that the microorganism has. Also, microorganisms that contribute to health of humans as hosts are also called "probiotics". Products such as supplements, yogurts, and creams using probiotics have been widely commercialized.

### CITATION LIST

### PATENT LITERATURE

PTL 1: WO 2017/195872 A1
PTL 2: WO 2021/040021 A1

### SUMMARY OF INVENTION

### TECHNICAL PROBLEM

The plurality of types of microorganisms as described above cohabitate while exchanging metabolites and interacting with each other. In recent years, studies for revealing a symbiosis mechanism of a plurality of types of microorganisms, evaluating medical efficacy for the plurality of types of microorganisms, and the like have been advanced. Therefore, technologies that enable suitable analysis of interactions among the plurality of types of microorganisms have been required.

As a method of preparing a plurality of types of microorganisms as analysis targets, mixed culture is known. However, since microorganisms are in a floating state in a liquid sample and there are far distances between microorganisms in the mixed culture in general, interactions among the microorganisms are less likely to occur. Thus, fixing the microorganisms at a high density, in other words, accumulating the microorganisms is conceivable. Since interactions among the microorganisms thus become likely to occur, it is possible to expect an improvement in analysis accuracy.

A microorganism collecting device disclosed in WO 2017/195872 A1 (PTL 1) collects a plurality of microorganisms dispersed in a liquid sample. The collecting device includes a light source and a holding member configured to hold the liquid sample. A photothermal conversion region is formed in the holding member. The photothermal conversion region causes heat convection in the liquid sample by converting light from the light source into heat and heating the liquid sample. It enables to accumulate the microorganisms by using the heat convection.

Applying the device disclosed in PTL 1 to accumulation of a plurality of types of microorganisms is also conceivable. However, the microorganisms are also heated with the heating of the liquid sample. Therefore, the microorganisms may be thermally damaged, and the microorganisms may die out. Then, it may not be possible to suitably analyze interactions among the plurality of types of microorganisms.

The present disclosure has been made to solve the above problem, and an object thereof is to provide a microorganism accumulation method and an accumulation system suitable for analyzing interactions among a plurality of types of microorganisms.

### SOLUTION TO PROBLEM

According to an aspect of the present disclosure, a microorganism accumulation method is for accumulating a plurality of types of microorganisms included in a liquid sample. The microorganism accumulation method includes first to fourth steps. The first step is preparing a substrate provided with a plurality of pores. Each of the plurality of pores includes an opening that is capable of capturing at least one microorganism for each of the plurality of types and a depth that extends in a direction including a vertically downward component. The second step is introducing the liquid sample onto the substrate. The third step is setting an irradiation condition for non-resonant light that is light outside an electronic resonance wavelength range of the plurality of types of microorganisms. The fourth step is irradiating the plurality of pores with the non-resonant light through the liquid sample in accordance with the irradiation condition. A region irradiated with the non-resonant light in the plurality of pores does not include a photothermal conversion material that converts the non-resonant light into heat. The setting includes setting, for the plurality of types of microorganisms, intensity of the non-resonant light within an irradiation range of the non-resonant light such that a magnitude of a vertically downward component of a light-induced force caused by irradiation with the non-resonant light (i) is greater than a magnitude of a vertically upward component of a buoyancy force caused by the liquid sample and (ii) is greater than a magnitude of a vertically upward component of a force caused by a Brownian motion of molecules in the liquid sample.

According to another aspect of the present disclosure, a microorganism accumulation system accumulates a plurality of types of microorganisms included in a liquid sample. The microorganism accumulation system includes a substrate that is provided with a plurality of pores. Each of the plurality of pores includes an opening that is capable of capturing at least one microorganism for each of the plurality of types and a depth that extends in a direction including a vertically downward component. The accumulation system further includes: a light source that irradiates the plurality of pores with non-resonant light through the liquid sample in a state where the liquid sample is disposed on the substrate, the non-resonant light being light outside an electronic resonance wavelength range of the plurality of types of microorganisms; and a controller that controls the light source. A region irradiated with the non-resonant light in the plurality of pores does not include a photothermal conversion material that converts the non-resonant light into heat. The controller sets, for the plurality of types of microorganisms, intensity of the non-resonant light within an irradiation range of the non-resonant light such that a magnitude of a vertically downward component of a light-induced force caused by irradiation with the non-resonant light (i) is greater than a magnitude of a vertically upward component of a buoyancy force caused by the liquid sample and (ii) is greater than a magnitude of a vertically upward component of a force caused by a Brownian motion of molecules in the liquid sample.

### ADVANTAGEOUS EFFECT OF INVENTION

According to the present disclosure, it is possible to suitably analyze interactions among a plurality of types of microorganisms.

### BRIEF DESCRIPTION OF DRAWINGS

Fig. 1 is an overall configuration diagram of a microorganism accumulation system according to an embodiment of the present disclosure.
Fig. 2 is an exploded perspective view of an accumulation kit.
Fig. 3A is a top view of the accumulation kit. Fig. 3B is a sectional view of the accumulation kit along the line IIIB-IIIB in Fig. 3A.
Fig. 4 is an image diagram illustrating how a plurality of types of microorganisms are accumulated.
Fig. 5 is a flowchart illustrating a processing procedure for a manufacturing method of the accumulation kit according to the present embodiment.
Figs. 6A to 6E are schematic process diagrams (first to fifth diagrams) of the manufacturing method of the accumulation kit according to the present embodiment.
Fig. 7 is an SEM image illustrating an example of a mold.
Fig. 8 is a diagram illustrating a top image of an accumulation substrate.
Fig. 9 is a diagram illustrating a sectional image of the accumulation substrate.
Fig. 10 is a diagram for explaining a light-induced force acting on microorganisms through irradiation with laser light from a laser light source.
Fig. 11 is a diagram for explaining a mechanism of accumulating a plurality of types of microorganisms according to the present embodiment.
Fig. 12 is a flowchart illustrating a processing procedure for microorganism accumulation processing.
Fig. 13 is a diagram for explaining a relationship between a light pressure and a spot diameter.
Fig. 14 is an SYTO9 image indicating microorganism accumulation results for different spot diameters.
Fig. 15 is a diagram illustrating a relationship between the spot diameter and the area of a region where fluorescence is observed in the fluorescent image in Fig. 14.
Fig. 16 is an SYTO9 image indicating microorganism accumulation results for different light irradiation times.
Fig. 17 is a PI image indicating a microorganism accumulation result for a light irradiation time of 60 minutes.
Fig. 18 is a diagram illustrating a relationship among a light irradiation time, a survival rate, and a fluorescent area.
Fig. 19 is an SYTO9 image indicating microorganism accumulation results for different laser power.
Fig. 20 is a diagram illustrating a relationship among laser power, a survival rate, and an accumulation density.
Fig. 21 is a fluorescent observation image indicating an accumulation result of a plurality of types of microorganisms stained with multiple colors.
Fig. 22 illustrates fluorescent observation images indicating bacteria accumulation results in a high-concentration sample.
Fig. 23 illustrates fluorescent observation images indicating bacteria accumulation results in a low-concentration sample.
Fig. 24 is an enlarged optical image to observe accumulated bacteria.
Fig. 25 is a diagram illustrating an accumulation area and a survival rate of the bacteria.
Fig. 26 is an overall configuration diagram of a microorganism accumulation system according to a modification of the embodiment of the present disclosure.
Fig. 27 is a diagram illustrating an example of a mold to form a microchannel substrate.
Fig. 28 is a diagram illustrating the microchannel substrate produced by using the mold illustrated in Fig. 27.
Fig. 29 is a diagram illustrating an example of an accumulation substrate.
Fig. 30 is an image of an actually produced accumulation kit.
Fig. 31 is a diagram for explaining a mechanism of accumulating a plurality of types of microorganisms according to the modification.

### DESCRIPTION OF EMBODIMENT

### <Description of terms>

In the present disclosure and an embodiment thereof, "sub-micrometer order" includes a range of 100 nm (= 0.1 µm) to 1000 nm (= 1 µm). "Micrometer order" includes a range of 1 µm to 1000 µm (= 1 mm). Therefore, "from a sub-micrometer order to a micrometer order" includes a range of 0.1 µm to 1000 µm. The term "from a sub-micrometer order to a micrometer order" may preferably mean a range of several hundreds of nm to several hundreds of µm, and may more preferably mean a range of 1 µm to several tens of µm.

In the present disclosure and the embodiment thereof, "microorganisms" mean organisms having sizes within a range of a sub-micrometer order to a micrometer order. The shapes of the microorganisms are not particularly limited and are, for example, spherical shapes, elliptical spherical shapes, and rod shapes (pole shapes). In a case where a microorganism has an elliptical spherical shape, it is only necessary for at least one of the length of the elliptical sphere in a long-axis direction and the length thereof in a short-axis direction to fall within the range of a sub-micrometer order to a micrometer order. In a case where a microorganism has a rod shape, it is only necessary for at least one of the width and the length of the rod to fall within the range of a sub-micrometer to a micrometer order.

All unicellular organisms are microorganisms. Some of multicellular organisms are also microorganisms. More specifically, organisms are categorized into bacteria, archaea, and eukaryotes. Most of the bacteria and the archaea are unicellular organisms and are thus microorganisms. Species that exhibit multicellularity among the bacteria and the archaea are also microorganisms. Most of the eukaryotes also exhibit unicellularity and are thus microorganisms. Microorganisms may also include human cells and the like.

In the present disclosure and the embodiment thereof, a "light-induced force" is a collective term of a dissipative force, a gradient force, and an inter-object light-induced force. The dissipative force is a force generated by the momentum of light being given to a substance in a dissipative process such as light scattering or light absorption. The gradient force is a force that causes a substance that has caused light-induced polarization to move to a stabilization point of an electromagnetic potential in a case where the substance is placed in a non-uniform electromagnetic field. The inter-object light-induced force is a sum of a force due to a longitudinal electric field and a force due to a lateral electric field (radiation field) caused by induced polarization in a plurality of photoexcited substances.

In the present disclosure and the embodiment thereof, "visible light" means light in a wavelength range of 400 nm to 700 nm. "Infrared light" means light in a wavelength range of 700 nm to 10,000 µm (= 1 mm), preferably means light in a wavelength range of 700 nm to 2,500 nm, and more preferably means light in a wavelength range of 700 nm to 1,400 nm. "White light" means light in a wavelength range from an ultraviolet range to a near-infrared range (for example, a wavelength range of 200 nm to 1100 nm).

In the present disclosure and the embodiment thereof, "resonant light" means light that causes large light-induced polarization in microorganisms through incidence on the microorganisms. The light-induced polarization is electric polarization caused by electrons inside a substance being excited by light. The resonant light has a wavelength in an electronic resonance wavelength range of the microorganisms. On the other hand, "non-resonant light" means light that causes small light-induced polarization in the microorganisms through incidence on the microorganisms. The non-resonant light has a wavelength outside the electronic resonance wavelength range of the microorganisms.

In the present disclosure and the embodiment thereof, "chemotaxis" means a characteristic that the microorganisms show directional responses to concentration gradients of specific chemical substances that are present around the microorganisms. The microorganisms having chemotaxis show responses of approaching preferable chemical substances and retreating from unfavorable chemical substances.

### [Embodiment]

Hereinafter, an embodiment of the present disclosure will be described in detail with reference to the drawings. In the drawings, the same or corresponding parts will be denoted by the same reference signs, and description thereof will not be repeated. An x direction and a y direction represent a horizontal direction. The x direction and the y direction perpendicularly intersect each other. A z direction represents a vertical direction. The gravity is directed downward in the z direction. The upper side in the z direction (vertically upward) will be abbreviated as an "upper side" or "upward", and the lower side in the z direction (vertically downward) will be abbreviated as a "lower side" or "downward".

### <System overall configuration>

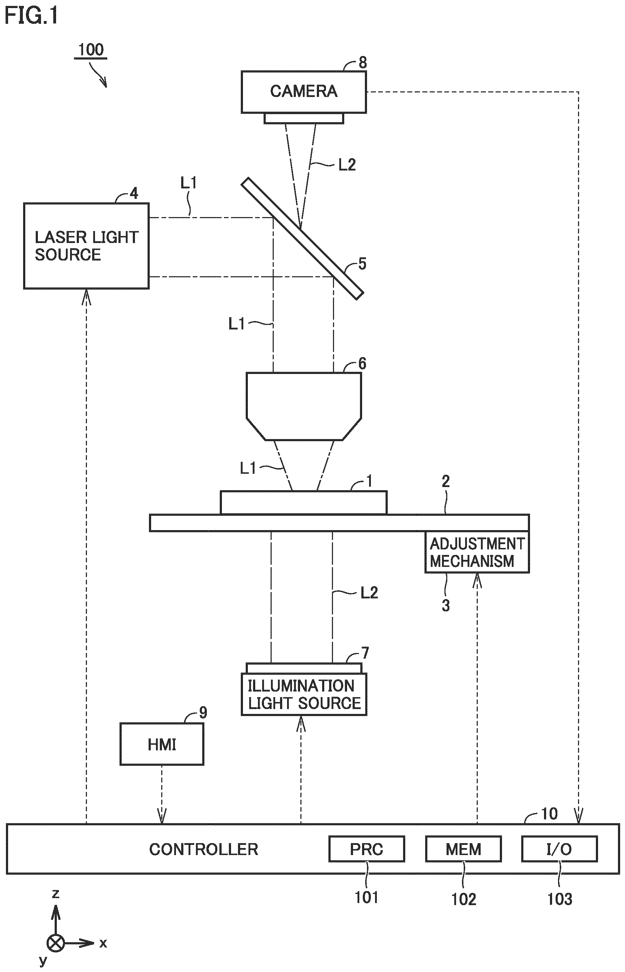

Fig. 1 is an overall configuration diagram of a microorganism accumulation system according to an embodiment of the present disclosure. An accumulation system 100 includes an accumulation kit 1, an XYZ stage 2, an adjustment mechanism 3, a laser light source 4, a dichroic mirror 5, an objective lens 6, an illumination light source 7, a camera 8, a human machine interface (HMI) 9, and a controller 10.

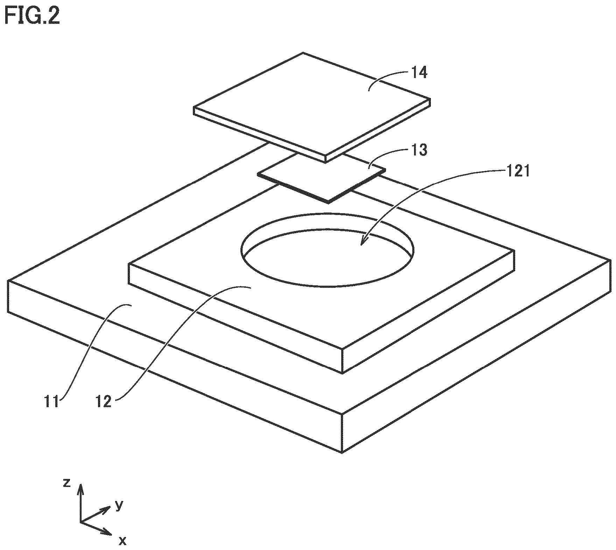

Accumulation kit 1 holds a sample. The sample is a liquid sample including a plurality of types of microorganisms as accumulation targets. Although the type of the liquid (a dispersion medium of the plurality of types of microorganisms) is not particularly limited, the liquid is sterilized water in this example. A configuration example of accumulation kit 1 will be described in Figs. 2 to 9.

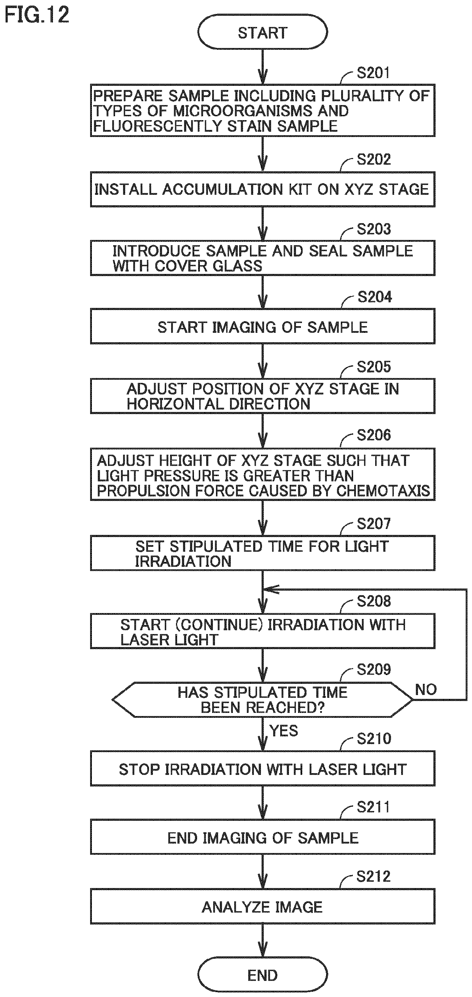

XYZ stage 2 holds accumulation kit 1. XYZ stage 2 is provided with a through-hole (not illustrated) to transmit white light (which will be described later) therethrough. Although not illustrated, a plurality of accumulation kit may be prepared. In such a case, a plurality of accumulation kits 1 are installed on XYZ stage 2 in order, and accumulation processing (see Fig. 12), which will be described later, is performed on each accumulation kit 1.

Adjustment mechanism 3 is a drive mechanism such as a servo motor or a focusing handle. Adjustment mechanism 3 adjusts the position of XYZ stage 2 in the horizontal direction and the height thereof in the vertical direction in accordance with a command from controller 10. It is thus possible to adjust a relative positional relationship between accumulation kit 1 and objective lens 6.

Laser light source 4 emits laser light (indicated by L1) of a continuous wave (CW) in accordance with a command from controller 10. It is also possible to adjust power (unit: W) of the laser light by a command from controller 10. The wavelength of the laser light is outside an electronic resonance wavelength range of the microorganisms. In the present embodiment, the wavelength of the laser light is a wavelength of a near-infrared range (specifically, 1064 nm). The laser light emitted from laser light source 4 is directed to dichroic mirror 5.

Dichroic mirror 5 is disposed between laser light source 4 and objective lens 6 and between objective lens 6 and camera 8. Dichroic mirror 5 is configured to transmit visible light while reflecting infrared light. Therefore, the laser light that is near-infrared light is reflected by dichroic mirror 5.

Objective lens 6 collects the laser light reflected by dichroic mirror 5. Accumulation kit 1 is irradiated with the laser light collected by objective lens 6.

Illumination light source 7 is an artificial light source such as a halogen lamp, a mercury lamp, a light-emitting diode (LED), or a fluorescent light. Illumination light source 7 emits white light (indicated by L2) to image the sample on accumulation kit 1 in accordance with a command from controller 10. Accumulation kit 1 is irradiated with the white light after passing through the through-hole provided in XYZ stage 2, and the white light is then transmitted through accumulation kit 1. The white light transmitted through accumulation kit 1 is directed to dichroic mirror 5 via objective lens 6. The white light that is visible light is transmitted through dichroic mirror 5 and is taken by camera 8.

Camera 8 includes an imaging element such as a charge coupled device (CCD) image sensor or a complementary metal oxide semiconductor (CMOS) image sensor. Camera 8 captures an image of the sample on accumulation kit 1 and outputs a signal indicating the captured image to controller 10 in accordance with a command from controller 10. The image captured by camera 8 may be a still image or a moving image.

HMI 9 includes, for example, input devices such as a switch, a mouse, a keyboard, and a touch panel and output devices such as a lamp, a display, and a speaker (both of which are not illustrated). HMI 9 receives a user's operation and outputs a signal indicating the user's operation to controller 10. Note that the user described herein is a person who is in charge of handling the microorganisms and is a developer, a researcher, a student, a technician, an operator, or the like.

Controller 10 includes a processor 101 such as a central processing unit (CPU), a memory 102 such as a read only memory (ROM) and a random access memory (RAM), and an input/output port 103 to and from which various signals are input and output. Controller 10 controls each of devices (adjustment mechanism 3, laser light source 4, illumination light source 7, and camera 8) in accumulation system 100. Moreover, controller 10 can also perform image analysis (fluorescent image analysis in an example, which will be described later) on the image captured by camera 8.

Note that the optical system illustrated in Fig. 1 is an example. The optical system in accumulation system 100 may include other optical components (such as a mirror, a half mirror, a beam splitter, a filter, and an optical fiber) instead of or in addition to dichroic mirror 5. The optical system illustrated in Fig. 1 is configured such that the sample is irradiated from the lower side to the upper side with the white light from illumination light source 7 and camera 8 on the upper side images the sample on the lower side. However, the optical system in accumulation system 100 may be configured such that the sample is irradiated from the lower side to the upper side with the white light from illumination light source 7 and camera 8 on the lower side images the sample on the upper side.

Laser light source 4 corresponds to the "light source" according to the present disclosure. The laser light emitted from laser light source 4 corresponds to the "non-resonant light" according to the present disclosure. Controller 10 corresponds to the "controller" according to the present disclosure.

### <Configuration of accumulation kit>

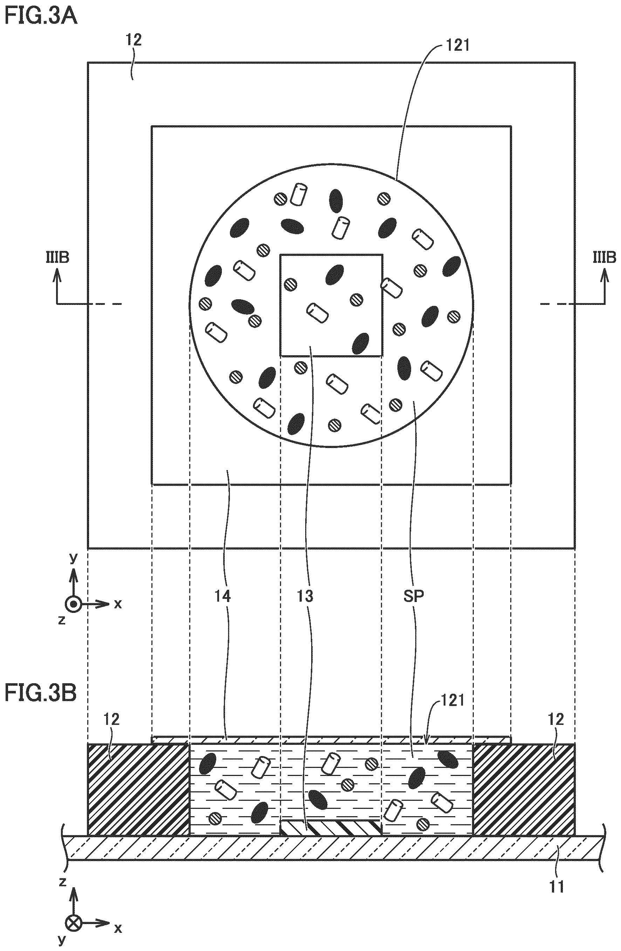

Fig. 2 is an exploded perspective view of accumulation kit 1. Fig. 3A is a top view of accumulation kit 1. Fig. 3B is a sectional view of accumulation kit 1 along the line IIIB-IIIB in Fig. 3A. Referring to Figs. 2 to 3B, accumulation kit 1 includes a base substrate 11, a holding frame 12, an accumulation substrate 13, and a cover glass 14.

Base substrate 11 is provided to secure mechanical strength of accumulation kit 1. Furthermore, base substrate 11 is configured to transmit white light from illumination light source 7. Specifically, base substrate 11 is, for example, a glass substrate (slide glass). Base substrate 11 may be a silicone substrate, a polyethylene terephthalate (PET) film, or the like. Although the shape of base substrate 11 is not particularly limited, the shape of base substrate 11 is a planar shape (rectangular parallelepiped shape) in this example.

Holding frame 12 holds a sample (indicated by SP). In this example, holding frame 12 is provided with a columnar-shaped through-hole 121. Through-hole 121 functions as a "liquid reservoir" that accumulates a specific amount of sample.

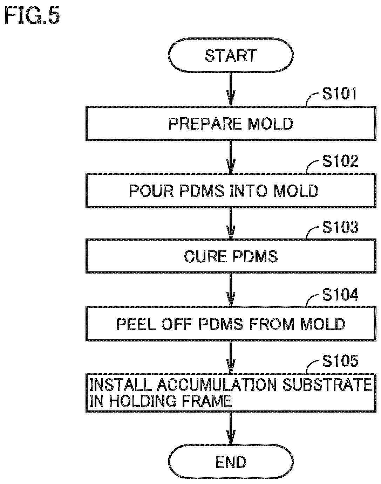

Accumulation substrate 13 is disposed inside through-hole 121 in holding frame 12. Accumulation substrate 13 is configured to accumulate a plurality of types of microorganisms with irradiation with laser light. A manufacturing method and a detailed structure of accumulation substrate 13 will be described in Figs. 5 to 9.

Cover glass 14 covers, from the upper side, the sample held inside through-hole 121 in holding frame 12. As illustrated in Fig. 3B, it is desirable that through-hole 121 be filled with the sample and the sample be sealed with cover glass 14. A reason therefor will be described later.

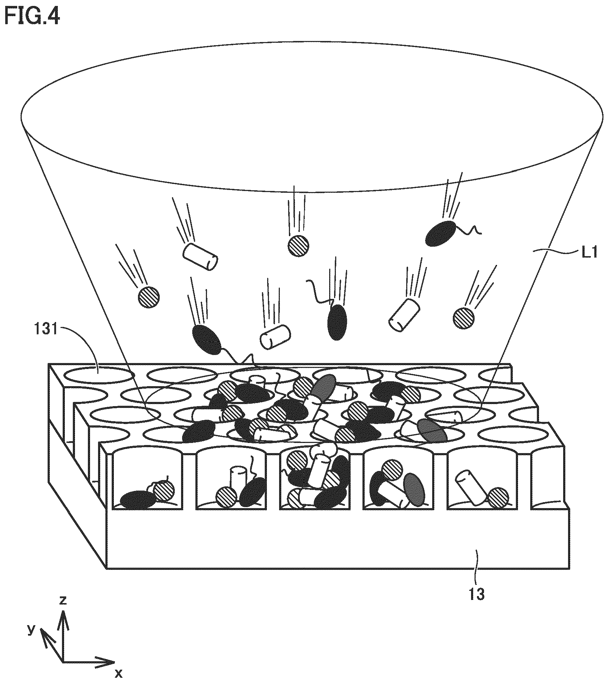

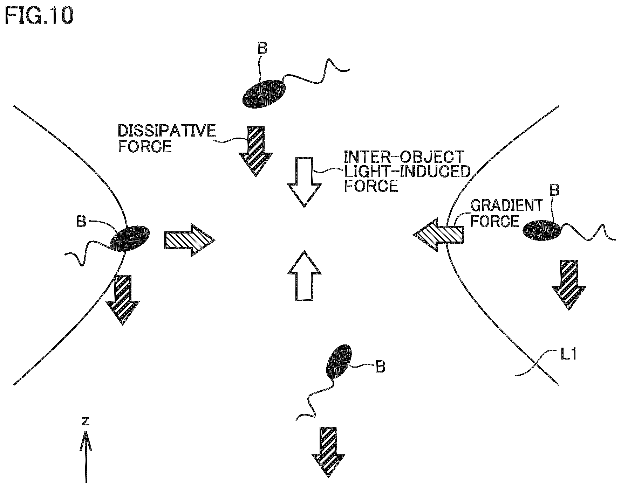

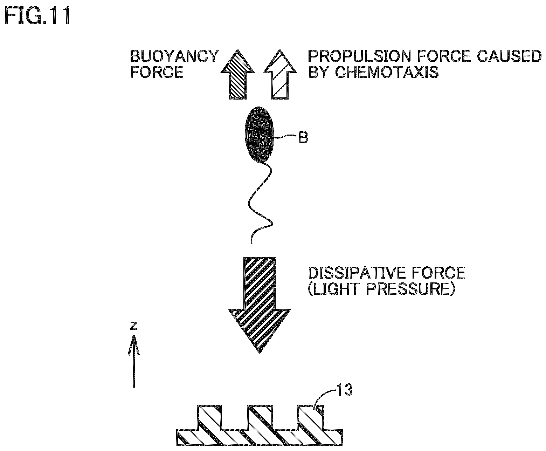

Fig. 4 is an image diagram illustrating how the plurality of types of microorganisms are accumulated. Accumulation substrate 13 is provided with a plurality of pores 131 aligned in a honeycomb shape. Each of plurality of pores 131 includes an opening that can capture at least one microorganism for each of the plurality of types and a depth that extends in a direction including a vertically downward component. According to the present embodiment, it is possible to accumulate the plurality of types of microorganisms floating in the sample in pores 131 in accumulation substrate 13 by irradiating the sample with the laser light from laser light source 4. The accumulation mechanism will also be described in Figs. 10 and 11.

Note that three types of microorganisms are schematically illustrated in samples in Figs. 3A, 3B, and 4. However, the number of types of microorganisms is not limited to three and may be any number as long as it is two or more. Also, typical microorganisms are too small to be able to be observed with naked eyes in practice.

### <Manufacturing flow of accumulation kit>

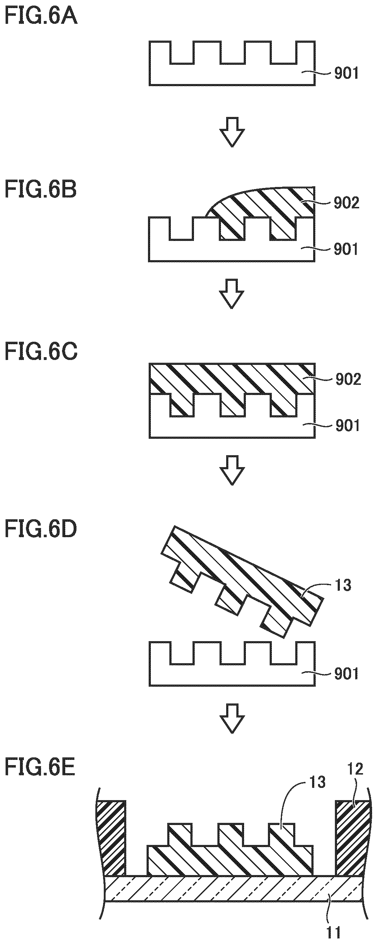

Fig. 5 is a flowchart illustrating a processing procedure for a manufacturing method of accumulation kit 1 according to the present embodiment. Figs. 6A to 6E are schematic process diagrams of the manufacturing method of accumulation kit 1 according to the present embodiment. Hereinafter, a step will be abbreviated as "S".

Referring to Figs. 5 and 6A to 6E, a mold (casting mold) 901 to manufacture accumulation substrate 13 is prepared in S101 (see Fig. 6A). For example, mold 901 can be produced by processing silicon using a technology such as electron beam lithography or dry etching.

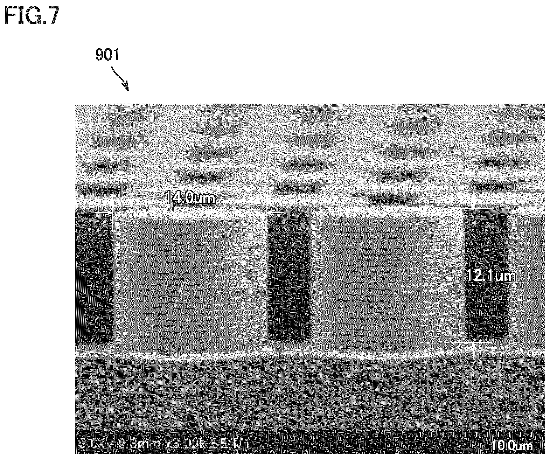

Fig. 7 is a scanning electron microscope (SEM) image illustrating an example of mold 901. Mold 901 includes a plurality of columnar-shaped protrusions aligned in a honeycomb shape, for example. The diameter and the height of the protrusions can be set to any values. In this example, the diameter of the protrusions is 14.0 µm, and the height of the protrusions is 12.1 µm.

Returning to Figs. 5 and 6A to 6E, dimethylpolysiloxane (PDMS) which is liquid silicone rubber is used as a material of accumulation substrate 13 in the present embodiment. In S102, a mixed solution 902 of a PDMS prepolymer (PDMS before curing) and a curing agent is poured into mold 901 (see Fig. 6B).

In S103, mixed solution 902 of the PDMS prepolymer and the curing agent after deaeration is baked under a predetermined condition, thereby curing PDMS (see Fig. 6C).

In S104, cured mixed solution 902 is peeled off from mold 901, thereby completing accumulation substrate 13 (see Fig. 6D).

In S105, accumulation substrate 13 is disposed in holding frame 12 (see Fig. 6E). In this manner, accumulation kit 1 before introduction of a sample is completed, and the series of processing is ended.

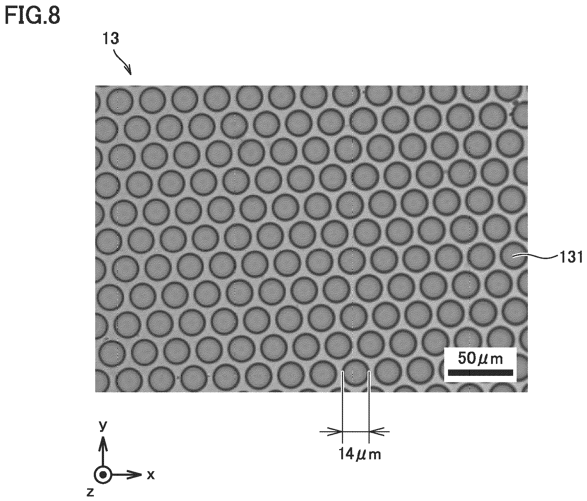

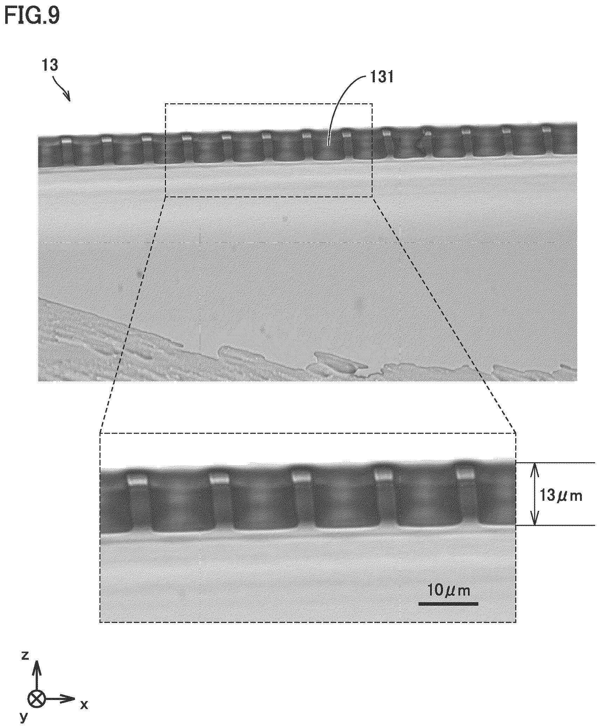

Fig. 8 is a diagram illustrating a top image of accumulation substrate 13. Fig. 9 is a diagram illustrating a sectional image of accumulation substrate 13. As illustrated in Figs. 8 and 9, accumulation substrate 13 is provided with a plurality of columnar-shaped pores 131 aligned in a honeycomb shape. The diameter of pores 131 was 14 µm, and the depth of the pores was 13 µm.

### <Accumulation mechanism>

Subsequently, an accumulation mechanism of a plurality of types of microorganisms according to the present embodiment will be described. In each example, which will be described later, three types of bacteria, specifically, lactobacillus casei, pseudomonas aeruginosa, and staphylococcus aureus were used as microorganisms as accumulation targets.

Lactobacillus casei is a rod-shaped bacterium. The length (long diameter) of a long axis of typical lactobacillus casei is about 1 µm to 3 µm. Pseudomonas aeruginosa is a rod-shaped bacterium. The length of a long axis of typical pseudomonas aeruginosa is about 0.7 µm to 2 µm. Staphylococcus aureus is a spherical bacterium. The diameter of typical staphylococcus aureus is about 0.8 µm to 1 µm. Lactobacillus casei and pseudomonas aeruginosa have chemotaxis. On the other hand, staphylococcus aureus does not have chemotaxis.

Note that intestinal bacteria are generally categorized into good bacteria, bad bacteria, and opportunistic bacteria. Lactobacillus casei is a good bacterium. Pseudomonas aeruginosa is a bad bacterium. Staphylococcus aureus is an opportunistic bacterium. It is possible to set a ratio of good bacteria, bad bacteria, and opportunistic bacteria to a desired value by adjusting a content ratio of lactobacillus casei, pseudomonas aeruginosa, and staphylococcus aureus. For example, it is possible to create an ideal balance for intestinal bacteria flora (intestinal flora) of good bacteria:bad bacteria:opportunistic bacteria = 2:1:7.

Fig. 10 is a diagram for explaining a light-induced force acting on the bacteria through irradiation with laser light from laser light source 4. Fig. 10 illustrates, as an example, pseudomonas aeruginosa (indicated by B) that has flagella and has strong chemotaxis (active motility).

The light-induced force includes an inter-object light-induced force, a gradient force, and a dissipative force. The inter-object light-induced force and the gradient force particularly strongly act on the bacteria in the vicinity of a beam waist of the laser beam. In this manner, the bacteria are attracted in the direction of the beam waist. At a location separated from the vicinity of the beam waist, the dissipative force is significant as a light-induced force acting on the bacteria. A direction in which the dissipative force acts is the same direction as the irradiation direction of the laser light. Since the irradiation with the laser light is directed from the upper side to the lower side in this example, a dissipative force from the upper side to the lower side acts on the bacteria.

Note that the dissipative force includes a component based on light absorption (absorption force) and a component based on light scattering (scattering force). The absorption force is proportional to the absorption sectional area of a target object and laser intensity (= power/irradiation area). The scattering force is proportional to the scattering sectional area of the target object and the laser intensity. In the present embodiment, non-resonant light (laser light with a wavelength positioned outside the electronic resonance wavelength range of the plurality of types of bacteria) is employed as described above. This is because a light absorption range of bacteria is avoided to prevent the laser light itself from damaging the bacteria. Thus, the absorption force included in the dissipative force is negligibly small. Therefore, the dissipative force may be expressed as scattering force instead.

The size of each bacterium is about several µm. This is equivalent to the laser light wavelength of 1064 nm. Therefore, Mie scattering occurs if the bacteria are irradiated with the laser light, and the scattering force is still enhanced even under a condition that the light absorption range of bacteria is avoided (under the electronically non-resonant condition). More specifically, although a buoyancy force acts upward and a Brownian motion (collision) of molecules in the sample acts on each of the bacteria in the liquid sample, it is possible to cause a downward scattering force that is greater than (the magnitude of the upward component of) the buoyancy force and is greater than the magnitude of an upward component of the force caused by the Brownian motion to act on each of the bacteria in the present embodiment. More preferably, the magnitude of the downward component of the scattering force is greater than the sum of (the magnitude of the upward component of) the buoyancy force and the magnitude of the upward component of the force caused by the Brownian motion. It is thus possible to strongly push each of the bacteria into each pore. The dissipative force or the scattering force caused by the irradiation with the laser light will also be described as a "light pressure" below for simplicity.

Fig. 11 is a diagram for explaining an accumulation mechanism of the plurality of types of bacteria according to the present embodiment. The bacteria themselves that have chemotaxis may cause a propulsion force which is directed from the lower side to the upper side as illustrated in Fig. 11 in addition to the buoyancy force and the force caused by the Brownian motion acting on the bacteria that have chemotaxis. In this case, since the bacteria move in a direction away from accumulation substrate 13, it may be more difficult to accumulate the bacteria on accumulation substrate 13. Thus, the light pressure acting downward on the bacteria through the irradiation with the laser light is set to be greater than the upward propulsion force caused by the chemotaxis of the bacteria in the present embodiment. In other words, the magnitude of the downward component of the light pressure is greater than (the upward component of) the buoyancy force, is greater than the magnitude of the upward component of the force caused by the Brownian motion, and is greater than the upward component of the propulsion force caused by the chemotaxis. More preferably, the magnitude of the downward component of the light pressure is greater than the sum of the magnitude of the upward component of the buoyancy force, the magnitude of the upward component of the force caused by the Brownian motion, and the magnitude of the upward component of the propulsion force caused by the chemotaxis. It is possible to realize the accumulation of the plurality of types of bacteria including the bacteria that have chemotaxis by setting the light pressure to a value that is large enough to overcome the propulsion force caused by the chemotaxis.

It may be possible to accumulate the bacteria to some extent without irradiating the sample including the plurality of types of bacteria with the laser light if a significantly long time is spent. However, it is not possible to accumulate the bacterium in a form suitable for analysis of interactions among the plurality of types of bacteria. More specifically, in a case where irradiation with the laser light is not performed, staphylococcus aureus that does not have chemotaxis is captured in the pores first, and lactobacillus casei and pseudomonas aeruginosa are then captured in the pores in the order from those with weakest chemotaxis. Thus, the plurality of types of bacteria are non-uniformly distributed in the pores. In this case, spatial variations may occur in the interactions among the plurality of types of bacteria, and it is thus not possible to state that this is suitable for the analysis of the interactions. On the contrary, in the present embodiment, it is possible to accumulate the plurality of types of bacteria in a state where the bacteria are uniformly distributed in the pores. Therefore, according to the present embodiment, it is possible to suitably analyze the interactions among the plurality of types of bacteria.

### <Bacteria accumulation flow>

Fig. 12 is a flowchart illustrating a processing procedure for bacteria accumulation processing. The processing illustrated in the flowchart is executed when a predetermined condition is established (for example, when the user operates a start button (not illustrated) in HMI 9). Although each step is basically realized by software processing performed by controller 10 (processor 101), some or all of the steps may be realized by hardware (electrical circuit) disposed in controller 10.

In S201, controller 10 installs accumulation kit 1 on XYZ stage 2. The processing can be realized by a feeding mechanism (not illustrated) of accumulation kit 1, for example. The user may manually install accumulation kit 1 instead of automatically performing the processing.

In S202, controller 10 introduces a sample including a plurality of types of bacteria into holding frame 12 of accumulation kit 1. The processing can be realized by a dispenser (not illustrated) capable of pouring a sample. The user may manually introduce the sample into accumulation kit 1.

In S203, controller 10 seals the introduced sample with cover glass 14. Although the processing can also be automated by the feeding mechanism, the user may manually install cover glass 14.

In S204, controller 10 starts to capture an image of the sample in accumulation kit 1. More specifically, controller 10 controls illumination light source 7 to start irradiation of accumulation kit 1 with white light and controls camera 8 to capture the image of accumulation kit 1 at a laser spot.

In S205, controller 10 performs position adjustment in the horizontal direction using adjustment mechanism 3 such that the laser light irradiation position (the position of the laser spot) is located on accumulation substrate 13. Controller 10 can adjust the position of the laser spot on accumulation substrate 13 by extracting accumulation substrate 13 from the image captured by camera 8, for example.

In S206, controller 10 performs height adjustment of accumulation kit 1 in the vertical direction using adjustment mechanism 3 such that the light pressure of the laser light is above the propulsion force caused by the chemotaxis. A relationship as will be described later is present between the light pressure and the diameter of the laser spot on the upper surface of accumulation substrate 13 (hereinafter, abbreviated as a "spot diameter").

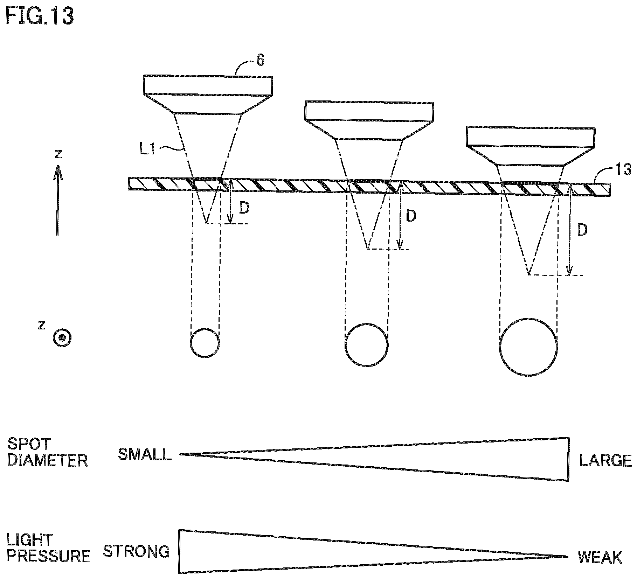

Fig. 13 is a diagram for explaining a relationship between the light pressure and the spot diameter. In the present embodiment, the height of accumulation kit 1 is adjusted such that the laser spot is located on the side further downward the upper surface of accumulation substrate 13. The distance between the laser spot and the upper surface of accumulation substrate 13 will be described as D.

The spot diameter increases as the distance D increases. In a case where power of the laser light is fixed, power of the laser light per unit area, that is, laser intensity (unit: W/m²) at the laser spot decreases and the light pressure thus decreases as the distance D increases. In this manner, a negative correlation is present between the light pressure and the spot diameter. It is possible to obtain the relationship among the distance D, the spot diameter, and the light pressure per unit area in previous calculation. Therefore, it is possible to set the light pressure to a desired value with which it is possible to overcome the propulsion force caused by the chemotaxis of the bacteria through the height adjustment (the adjustment of the distance D) of accumulation kit 1 in the vertical direction.

Referring again to Fig. 12, controller 10 sets the time length (stipulated time) during which irradiation with the laser light is to be performed in S207. Controller 10 receives a user's operation to set the stipulated time from HMI 9, for example. Alternatively, the stipulated time may be automatically set in accordance with the light pressure (the spot diameter and/or the height of accumulation kit 1 in the vertical direction). There is a trend that a shorter stipulated time is needed as the light pressure is longer. Controller 10 may have the relationship between the light pressure and the stipulated time as a map. Controller 10 can set the stipulated time corresponding to the light pressure set in S206 with reference to the map.

In S208, controller 10 controls laser light source 4 to start (or continue) to irradiate accumulation kit 1 with the laser light. The plurality of types of bacteria are accumulated in pores 131 in accordance with the mechanism described in Figs. 10 and 11 during the irradiation with the laser light.

In S209, controller 10 determines whether or not an elapse time from the start of the irradiation with the laser light has reached the stipulated time. In a case where the elapse time has not reached the stipulated time (NO in S209), controller 10 returns the processing to S208. This allows the irradiation with the laser light to be continued. Once the elapse time reaches the stipulated time (YES in S209), controller 10 moves on to the processing in S210 and controls laser light source 4 to stop the irradiation with the laser light. Furthermore, controller 10 ends the imaging of the sample in accumulation kit 1 (S211). In other words, controller 10 causes illumination light source 7 to stop the irradiation with the white light and causes camera 8 to stop the operation.

In S212, controller 10 analyzes the captured image of the sample (a fluorescent image in the example, which will be described later). Controller 10 may calculate the accumulation area of the bacteria and calculate a survival rate of the accumulated bacteria, for example. Thus, the series of processing is ended.

### <Comparison with related art>

Features of the accumulation mechanism of the plurality of types of bacteria in the present embodiment will be described in detail by comparing them with those of the mechanism described in PTL 1.

### <<1. Heating of laser spot>>

In the capturing and collecting kit described in PTL 1, a metal thin film is formed on a honeycomb polymer film. Once the metal thin film is irradiated with laser light, the metal thin film converts light energy into thermal energy, and the laser light irradiation position (laser spot) is locally heated (photo thermal effect). Then, a temperature gradient in which the temperature of the liquid sample increases at a position closer to the laser spot occurs, and heat convection is generated. Bacteria are accumulated in the vicinity of the laser spot by the bacteria being carried to the laser spot by the heat convection (see the paragraphs [0064] to [0072] in PTL 1). However, the bacteria are also heated with the heating of the liquid sample. As a result, the bacteria may be thermally damaged, and the bacteria may die out.

On the contrary, accumulation substrate 13 is not provided with a photothermal conversion material corresponding to the metal thin film in PTL 1 in the present embodiment. In the present embodiment, bacteria are accumulated by an optical method of pushing the bacteria into the pores with the light pressure set to be a value that is large enough to overcome the propulsion force caused by the chemotaxis. Therefore, according to the present embodiment, it is possible to accumulate a plurality of types of bacteria while suppressing thermal damage.

### <<2. Laser power and spot diameter>>

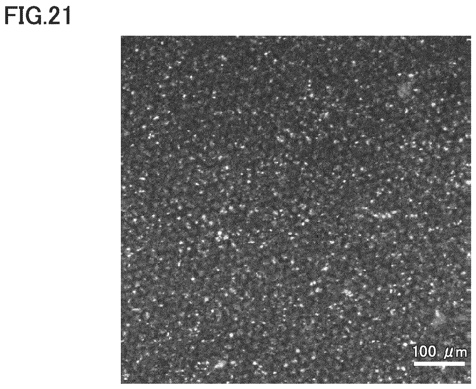

In PTL 1, the laser light is collected such that a partitioning wall that partitions the pores in the honeycomb polymer film can be irradiated therewith (see Figs. 12 and 21 and the paragraph [0103] in PTL 1). This is because collecting the laser light to the maximum extent is advantageous in terms of creating a large temperature gradient in the liquid sample. Also, the fact that it is possible to reduce thermal damage on the bacteria by limiting the region where heat is generated with the irradiation with the laser light (and a region in the surroundings to which the generated heat is transmitted) is also a reason therefor. Specific numerical values will be described. The laser light is collected such that the spot diameter is about several µm. Also, PTL 1 describes that the laser power from the laser light source is set to 40 mW. In PTL 1, the laser power after passing through an objective lens (magnification: 100×, oil immersion) is calculated as about 8 mW, which is about 20% of the laser power before passing through the objective lens (see the paragraph [0081] in PTL 1).

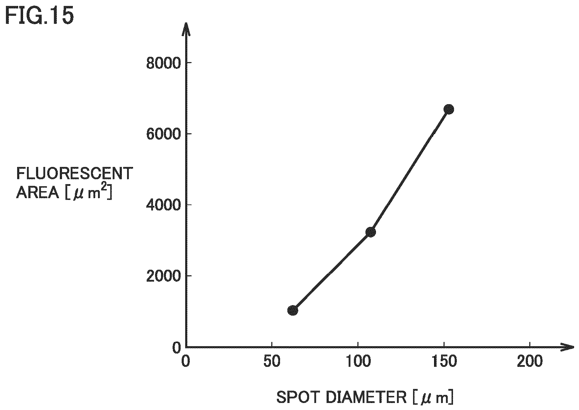

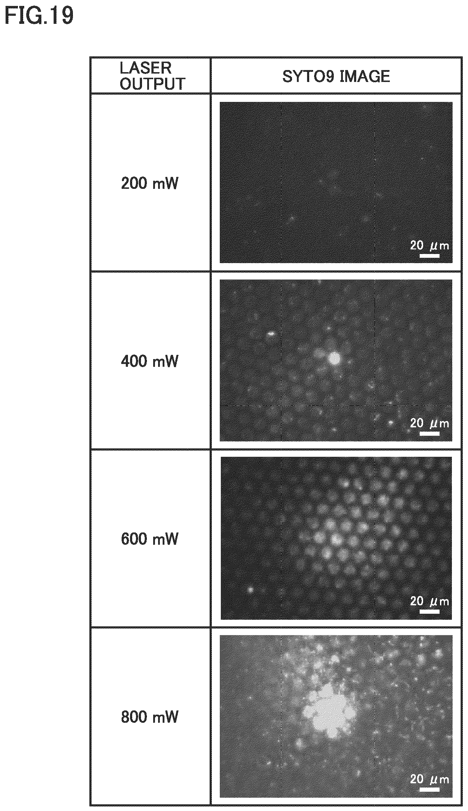

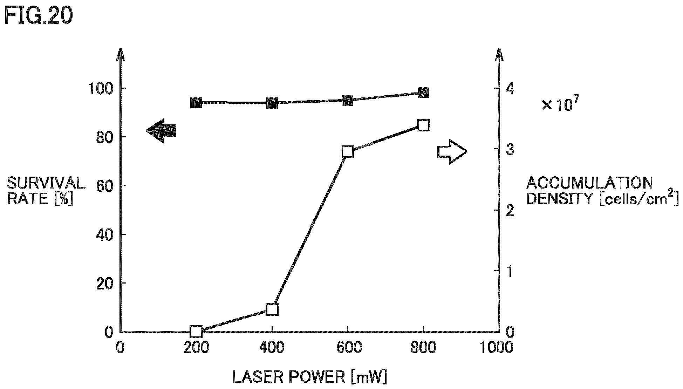

The bacteria accumulation mechanism in the present embodiment is to cause the light pressure of the laser light to act directly on the bacteria and push the bacteria into the pores. In order to increase the amount of accumulated bacteria, an increase in irradiation area of the sample with the laser light is required. Therefore, the laser spot is not extremely narrowed down in the present embodiment. In the example (see Fig. 15), which will be described later, the spot diameter is set within a range of 62.6 µm to 152.6 µm. Also, in the example (Figs. 19 and 20), which will be described later, the power of the laser light from laser light source 4 is set to 800 mW. The laser power after passing through the objective lens (magnification: 40×, dry) is calculated as about 320 mW, which is about 40% of the laser power before passing through the objective lens. The power value is a high value on the basis of which those skilled in the art cannot think that they will use the laser power to irradiate the microorganisms in accordance with the common technical knowledge.

In this manner, the laser light irradiation area is larger by two digits and the laser light power (unit: W) is larger by one to two digits (about 40 times) in the present embodiment than in PTL 1. In other words, a wide range is irradiated with the laser light with high intensity (unit: W/m²) in the present embodiment. In this manner, a strong light pressure that can overcome the propulsion force caused by the chemotaxis of the bacteria is applied over a wide range. It is thus possible to accumulate a large amount of bacteria with high efficiency.

### <<3. Pore size>>

In the capturing and collecting kit described in PTL 1, the pore diameter was about 5.0 µm, and the pore depth was about 3.0 µm (see Fig. 5 and the paragraph [0051] in PTL 1). As described above, the long axis lengths of lactobacillus casei, pseudomonas aeruginosa, and staphylococcus aureus are in a range of about 0.7 µm to 3 µm. In a case where the long axis lengths are 3 µm, for example, only several (two or three at the most) bacteria are captured in each pore provided in the capturing and collecting kit described in PTL 1.

On the contrary, the diameter of pores 131 was 14 µm, and the depth of pores 131 was 13 µm in the present embodiment. In other words, pores 131 provided in accumulation substrate 13 in the present embodiment are significantly larger than the pores provided in the capturing and collecting kit in PTL 1. Specifically, the opening area of pores 131 is 7.8 times. The depth of pores 131 is 4.3 times deeper. The volume of pores 131 is 34 times bigger. It is possible to accumulate a plurality of types of bacteria in the same pore by setting the size of each pore to be sufficiently large in this manner (by setting the size to be able to capture or accommodate at least one bacterium for each type). For example, it is possible to accumulate 68 to 102 bacteria with the long axis length of 3 µm. Therefore, it is possible to suitably analyze interactions among the plurality of types of bacteria.

### <<4. Communication between pores>>

In the capturing and collecting kit described in PTL 1, the honeycomb polymer film is formed by using, as molds, a plurality of water droplets aligned in a honeycomb shape through self-assembly (see Fig. 4 in PTL 1). Since each pore has a substantially spherical shape in the honeycomb polymer film due to the water droplets having a spherical shape, adjacent pores communicate with each other on the side of the bottom surface of the polymer film (see Fig. 5 and the paragraph [0052] in PTL 1). "Lateral holes" are formed in the capturing and collecting kit by the adjacent pores communicating with each other. Heat convection also flows in the direction (lateral hole direction) in which these lateral holes extend. The heat convection in the lateral hole direction plays a role in preventing the bacteria captured once in the pores from escaping from the pores.

On the contrary, in the present embodiment, adjacent pores do not communicate with each other, and "lateral holes" are not present as can be seen in the sectional view in Fig. 9. Therefore, heat convection flowing in the lateral hole direction is not generated. Thus, the pore depth is defined to prevent the bacteria from escaping due to chemotaxis even after the irradiation with the laser light is stopped in the present embodiment. Although the bacteria have chemotaxis, the bacteria just advance straight in the direction in which a concentration gradient of chemical substances (inducing substance/repellent substance) has occurred. Even if the bacteria advance straight in accordance with the concentration gradient of the chemical substances, it is possible to accommodate the movement within the pores by setting the pore depth to be sufficiently deep in consideration of the chemotaxis of the bacteria. It is thus possible to maintain the bacteria captured through light irradiation in a state where the bacteria are captured in the pores even after the light irradiation is stopped.

### <<5. Evaporation of dispersion medium>>

In the capturing and collecting kit described in PTL 1, a sample in a liquid droplet shape held on the capturing and collecting kit is opened toward an open space (see Fig. 3 in PTL 1). With evaporation of a dispersion medium in the sample from the liquid droplet surface, Marangoni convection may be generated in the sample. On the other hand, in PTL 1, buoyancy force convection with the irradiation with the laser light is actively used to accumulate the bacteria. Since the accumulation effect of the buoyancy force convection is strong, accumulation of the bacteria is not significantly prevented even if Marangoni convection occurs in addition to the buoyancy force convection.

On the contrary, the light pressure is used instead of the buoyancy force convection to accumulate bacteria in the present embodiment. Since the accumulation effect of the light pressure is not as strong as the accumulation effect of the buoyancy force convection, the Marangoni convection may prevent the accumulation of bacteria. Thus, through-hole 121 is filled with the sample, and the sample is sealed with cover glass 14 in the present embodiment (see Figs. 3A and 3B). It enables to suppress evaporation of the dispersion medium from the sample surface and to suppress occurrence of the Marangoni convection that may prevent accumulation of the bacteria by forming the closed system which the sample and the surrounding air are not in contact with each other in this manner. Also, since the distance between the sample surface and the laser spot is secured by increasing the volume of the dispersion medium, it is possible to reduce influences of the Marangoni convection.

### <Accumulation results>

Results of the accumulation processing for samples including the three types of bacteria (lactobacillus casei, pseudomonas aeruginosa, and staphylococcus aureus) will be described. All the bacteria were fluorescently stained. As a fluorescent dye, SYTO9 (registered trademark) or propidium iodide (PI) was used. SYTO9 stains both bacteria that are alive (living bacteria) and bacteria that are dead (dead bacteria). Once SYTO9 is externally excited, SYTO9 emits green fluorescent light. A fluorescent observation image based on an excitation waveform of SYTO9 will be described as an "SYTO9 image". On the other hand, PI stains only dead bacteria. Once PI is externally excited, PI emits red fluorescent light. A fluorescent observation image based on an excitation waveform of PI and also a "PI image" will be described.

### <<1. Spot diameter dependency>>

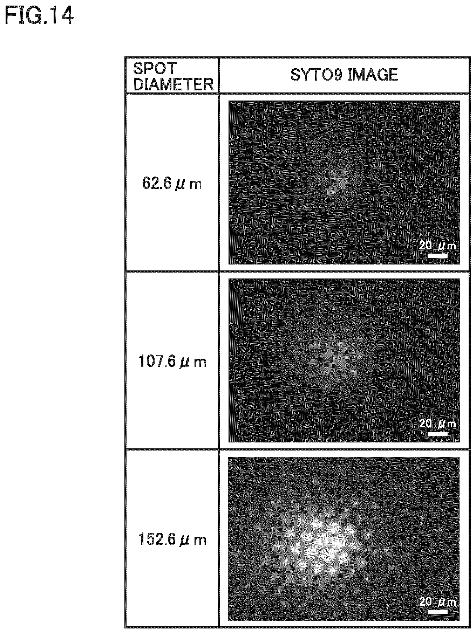

Fig. 14 is an SYTO9 image indicating accumulation results of bacteria (both living bacteria and dead bacteria) for different spot diameters. Fig. 15 is a diagram illustrating a relationship between the spot diameter and the area (fluorescent area) of a region where fluorescence is observed in the fluorescent image in Fig. 14. The horizontal axis of Fig. 15 represents the spot diameter. The vertical axis represents the fluorescent area (= bacteria accumulation area). The laser power from laser light source 4 was set to 800 mW. The light irradiation time (the stipulated time in Fig. 12) was set to 15 minutes. The concentration of bacteria was 10⁸ [CFU/mL] (colony forming unit: CFU).

In a case where the distance D between the laser spot and the upper surface of the accumulation substrate 13 was 40 µm, the spot diameter was 62.6 µm. In a case where the distance D was 70 µm, the spot diameter was 107.6 µm. In a case where the distance D was 100 µm, the spot diameter was 152.6 µm. As illustrated in Fig. 15, it was confirmed that the bacteria accumulation area increased as the distance D increased and the spot diameter thus increased along with that.

### <<2. Light irradiation time dependency>>

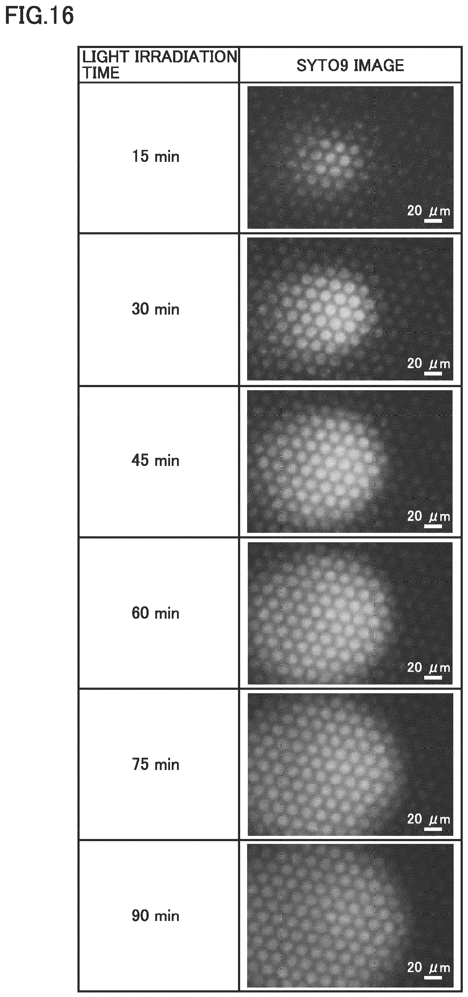

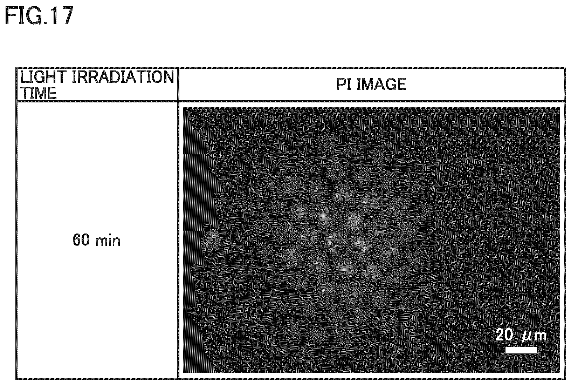

Fig. 16 is an SYTO9 image indicating accumulation results of bacteria (both living bacteria and dead bacteria) for different light irradiation times. Fig. 17 is a PI image indicating an accumulation result of bacteria (only dead bacteria) for a light irradiation time of 60 minutes. The light irradiation time was set every 15 minutes within a range of 15 minutes to 90 minutes. The spot diameter was set to 107.6 µm. The laser power was set to 800 mW. The concentration of bacteria was 10⁸ [CFU/mL].

It is possible to ascertain from Fig. 16 that the accumulation area of all the bacteria increases as the light irradiation time increases. It is possible to ascertain from Fig. 17 that a certain amount of bacteria may die out if the light irradiation time is as long as 60 minutes.

A survival rate of the accumulated bacteria can be calculated by analyzing the SYTO9 image and the PI image as illustrated in Figs. 16 and 17. The survival rate means the ratio of the number of living bacteria with respect to the total number of accumulated bacteria as indicated by Equation (1) below. Survival rate = the number of living bacteria/(the number of living bacteria + the number of dead bacteria) × 100 ...(1)

The denominator in the right side of Equation (1) is the number of bacteria observed in the SYTO9 image. The numerator in the right side is calculated by subtracting the number of bacteria observed in the PI image (= the number of dead bacteria) from the number of bacteria observed in the SYTO9 image (= the number of living bacteria + the number of dead bacteria).

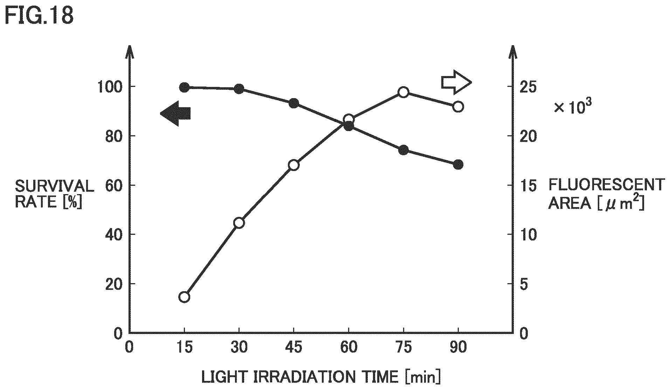

Fig. 18 is a diagram illustrating a relationship among the light irradiation time, the survival rate, and the fluorescent area. The horizontal axis represents the light irradiation time. The left vertical axis represents the survival rate of the bacteria. The right vertical axis represents the fluorescent area. In this example, the fluorescent area increased as the light irradiation time increased within a light irradiation time range of 15 minutes to 75 minutes. When the light irradiation time reached 75 minutes, the fluorescent area was saturated. No significant differences were observed in the fluorescent area between a case where the light irradiation time was 75 minutes and a case where the light irradiation time was 90 minutes. On the other hand, the survival rate monotonously decreased as the light irradiation time increased. However, the survival rate was still a high value exceeding 70% even when the light irradiation time was 90 minutes.

It is possible to ascertain from Fig. 18 that there is a light irradiation time that can achieve both a large fluorescent area and a high survival rate. In this example, it is possible to accumulate the bacteria with high efficiency while suppressing dying-out of the bacteria by setting the light irradiation time around 45 minutes.

PTL 1 describes that bacteria are accumulated in a light irradiation time that is as short as about 1 minute (see Fig. 12 and the paragraph [0074] in PTL 1). On the contrary, a longer light irradiation time of about ten and several minutes to several tens of minutes may be required in the present embodiment. However, according to the present embodiment, it is possible to accumulate bacteria while suppressing thermal damage in exchange for an increase in light irradiation time.

### <<3. Laser power dependency>>

Fig. 19 is an SYTO9 image indicating bacteria accumulation results for different laser power. The laser power was set to four values, namely 200 mW, 400 mW, 600 mW, and 800 mW. The spot diameter was set to 62.6 µm. The light irradiation time was set to 15 minutes. The concentration of bacteria was 10⁸ [CFU/mL]. Accumulation of bacteria was observed in the case where the laser power was 400 mW or more.

Fig. 20 is a diagram illustrating a relationship among the laser power, the survival rate, and the accumulation density. The horizontal axis represents the laser power. The left vertical axis represents the survival rate of the bacteria. The right vertical axis represents the accumulation density of the bacteria. It was confirmed that the bacteria was successfully accumulated at a high survival rate of over 90% and at a high density of 3.37 × 10⁷ [bacteria/cm²] in a case where the laser power was 800 mW.

### <<4. Multicolor staining>>

Fig. 21 is a fluorescent observation image indicating an accumulation result of a plurality of types of bacteria stained with multiple colors. Multicolor staining was performed by a fluorescence in situ hybridization (FISH) method. The spot diameter was set to 107.6 µm. The laser power was set to 800 mW. The light irradiation time was set to 5 minutes.

Specifically, lactobacillus casei was stained with a probe to which a green fluorescent dye (Alexa Flour (registered trademark) 488) was applied. The base sequence of the probe was 5'GGTATTAGCAYCTGTTTCCA3'. Pseudomonas aeruginosa was stained with a probe to which a yellow fluorescent dye (Alexa Flour 555) was applied. The base sequence of the probe was 5'TCTGGAAAGTTCTCAGCA3'. Staphylococcus aureus was stained with a probe to which a blue fluorescent dye (Alexa Flour 405) was applied. The base sequence of the probe was 5'GAAGCAAGCTTCTCGTCCGTTC3'.

Although it is not clear in the monochrome image in Fig. 21, fluorescence of the above three colors was observed. In this manner, it was confirmed that the three types of bacteria were successfully accumulated.

### <<5. Accumulation at high concentration>>

Accumulation results in high concentration/low concentration samples will be described. Specifically, 10⁹ [CFU/mL] was set as a high concentration, and 10⁸ [CFU/mL] was set as a low concentration. The low concentration was a concentration equivalent to the concentration in Figs. 14 to 20, and the high concentration was a concentration that was 10 times as high as the low concentration. The laser power was set to 800 mW. The spot diameter was set to 152.6 µm.

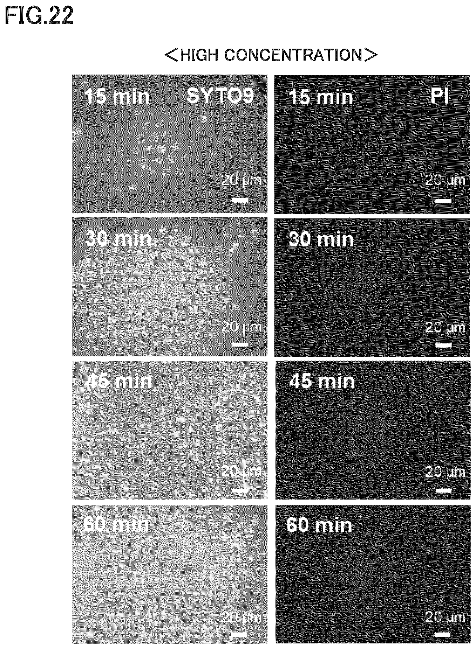

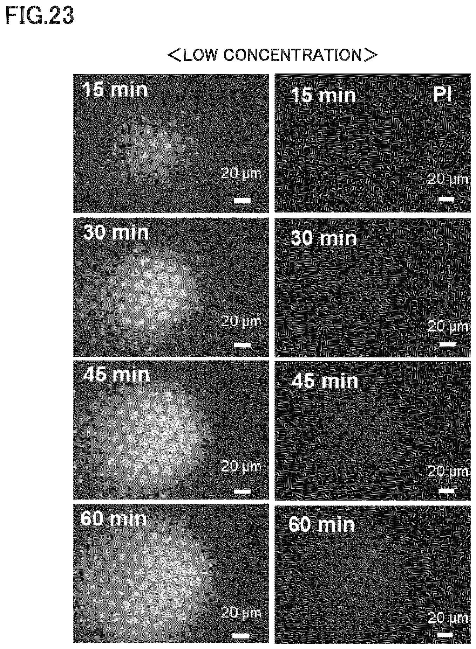

Fig. 22 illustrates fluorescent observation images indicating bacteria accumulation results in the high-concentration sample. Fig. 23 illustrates fluorescent observation images indicating bacteria accumulation results in the low-concentration sample. SYTO9 images are shown on the left side, and PI images are shown on the right side. It was clear that the bacteria accumulation area was wider and the amount of accumulated bacteria was larger in the high-concentration sample than in the low-concentration sample.

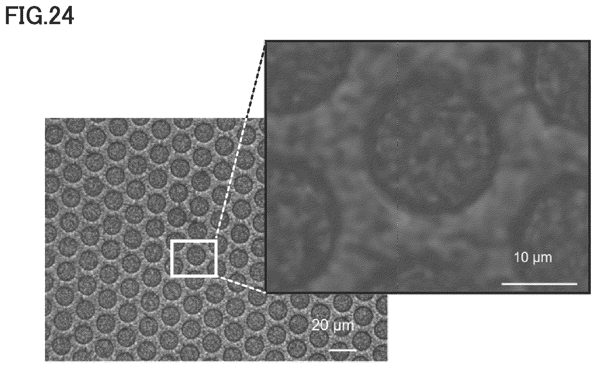

Fig. 24 is an enlarged optical image to observe accumulated bacteria. Since many bacteria captured in the pores and moving were observed, a high survival rate was expected.

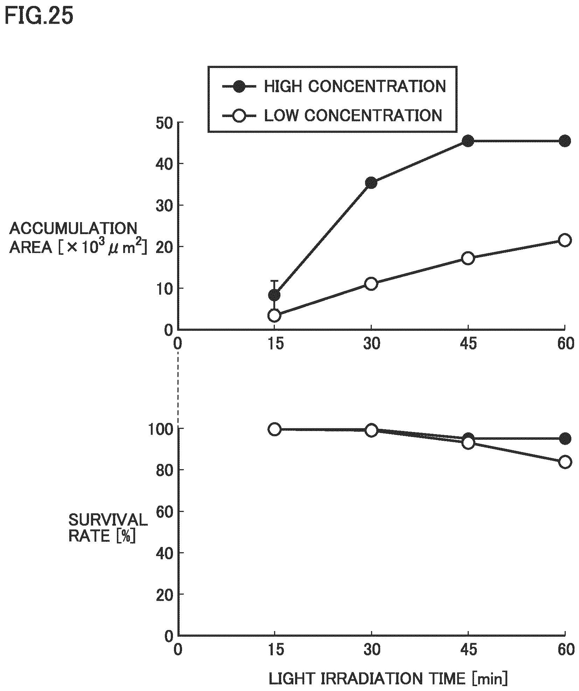

Fig. 25 is a diagram illustrating the bacteria accumulation area and the survival rate. The horizontal axis represents the light irradiation time. The upper vertical axis represents the bacteria accumulation area, and the lower vertical axis represents the survival rate of the bacteria. The rise of the accumulation area (increase rate) in the light irradiation time of 15 to 30 minutes, in particular, occurred more quickly in the high-concentration sample than in the low-concentration sample. Also, the accumulation area in the same light irradiation time was wider even after 30 minutes. A slight decrease in survival rate with elapse of time was observed in the low-concentration sample after the light irradiation time of 45 minutes. On the contrary, substantially no decrease in survival rate was observed in the high-concentration sample, and the survival rate in the high-concentration sample was a value that was almost 100%. The following two points are considered from the results illustrated in Figs. 22 to 25.

First, in the case of the high-concentration sample, more bacteria were pushed into and captured by the pores in an early stage through the irradiation with the laser light. The laser light was unlikely to fall directly on the bacteria captured in the pores. This is because the bacteria three-dimensionally overlap each other in the pores and arrival of the laser light at the bacteria in a lower layer was interrupted by the bacteria in an upper layer. Therefore, the bacteria captured in the pores are not damaged by the laser light at a considerable ratio. Therefore, it is considered to be possible to suppress a decrease in survival rate of the bacteria by increasing the concentration of the sample and capturing many bacteria in a short period of time.

Second, while the bacteria accumulation range was equivalent to the laser spot in the low-concentration sample (see Fig. 23), the bacteria accumulation range was significantly wider than the laser spot in the high-concentration sample (see Fig. 22). This means that bacteria that were not irradiated with light were also accumulated in the high-concentration sample. A reason therefor is considered to be because when bacteria irradiated with light were pushed toward the pores, a flow accompanying the movement of the bacteria occurred in the sample, and the surrounding bacteria that were not irradiated with the light were caught in the flow and were then captured in the pores.

Taking the above two considerations into consideration, then it is possible to ascertain that a high survival rate can be achieved by preparing the concentration of the sample to such a high concentration that bacteria are accumulated in a wider range than the laser spot.

As described above, the laser light irradiation condition is set such that a plurality of types of bacteria can be optically accumulated without using photothermal effect of a photothermal conversion material such as a metal thin film in the present embodiment. More specifically, a laser light irradiation range is preferably set to include the entire openings of two or more pores (see Fig. 4). Furthermore, the laser intensity (= power/irradiation area) is set such that the dissipative force (light-induced force) acting in the laser light irradiation direction is greater than the buoyancy force, is greater than the force caused by the Brownian motion, and is preferably greater than the propulsion force caused by chemotaxis (see Fig. 11). It thus enables to accumulate the plurality of types of bacteria in a living state in the pores over a wide range. Therefore, according to the present embodiment, it is possible to suitably analyze interactions among a plurality of types of bacteria including at least one type of bacteria that have chemotaxis.

Note that the example in which (the main surface of) accumulation substrate 13 is disposed in the horizontal direction has been described in the present embodiment. In this case, each of plurality of pores 131 has a depth that extends in the direction including only the vertically downward component. However, accumulation substrate 13 may be disposed with an inclination at a certain angle with respect to the horizontal direction. In other words, the depth of each of plurality of pores 131 may extend in a direction including a horizontal component in addition to the vertically downward component.

Moreover, the example in which the irradiation with the laser light from laser light source 4 is performed perpendicularly to (the main surface of) accumulation substrate 13 has been described in the present embodiment. In this case, a laser light propagation direction and the depth direction of plurality of pores 131 coincide with each other. However, irradiation with the laser light may be performed at an angle (except for a parallel angle) other than the angle perpendicular to accumulation substrate 13. In other words, the laser light propagation direction may not completely coincide with the depth direction of plurality of pores 131, and it is only necessary for the laser light propagation direction to include the vertically downward component.

### [Modification of embodiment]

In the embodiment, the example in which bacteria are accumulated in stationary samples has been described. In the present modification, accumulation of bacteria in a sample flowing through a microchannel will be described.

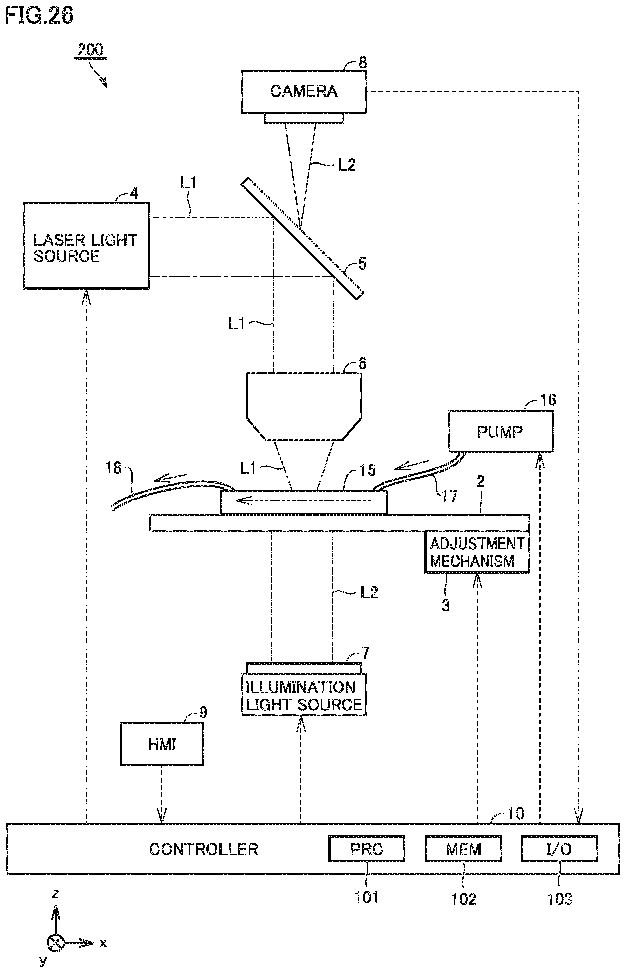

Fig. 26 is an overall configuration diagram of a microorganism accumulation system according to a modification of the embodiment of the present disclosure. An accumulation system 200 is different from accumulation system 100 (see Fig. 1) according to the embodiment in that accumulation system 200 includes an accumulation kit 15 instead of accumulation kit 1 and further includes a pump 16 and capillaries 17 and 18. The other configurations are the same.

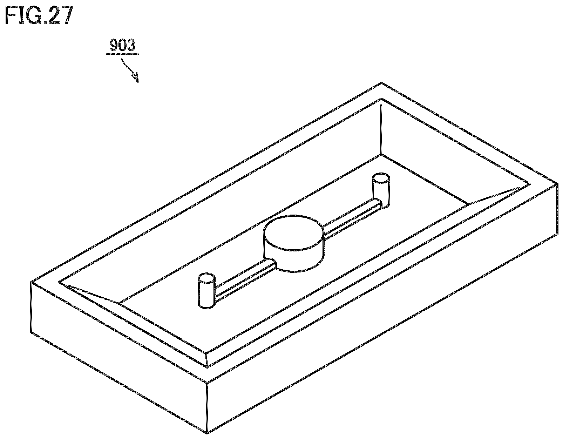

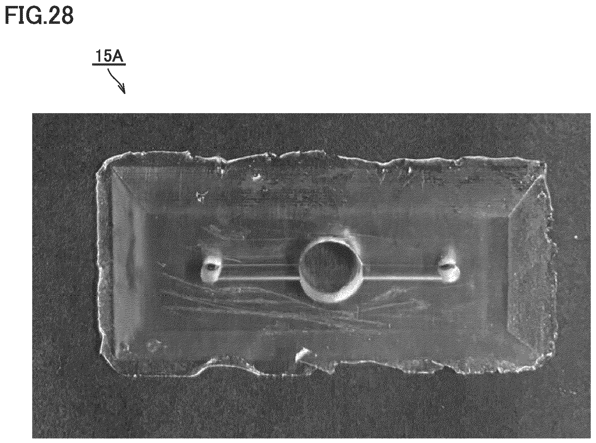

Accumulation kit 15 includes a microchannel substrate 15A (see Fig. 28). Accumulation kit 15 may be produced using a mold as in the embodiment. A configuration of accumulation kit 15 will be described in Figs. 27 to 30.

Pump 16 is configured to feed a sample by an effect such as a pressure, a centrifugal force, or a rotation force. Pump 16 may be, for example, an electric syringe pump or a manual dispenser or micropipette. Pump 16 is connected to capillary 17 provided on the upstream side of accumulation kit 15. The sample is distributed through accumulation kit 15 via capillary 17 and is then discharged from capillary 18 provided on the downstream side of accumulation kit 15.

Fig. 27 is a diagram illustrating an example of a mold to form the microchannel substrate. A mold 903 is produced using a 3D printer, for example.

Fig. 28 is a diagram illustrating the microchannel substrate produced using mold 903 illustrated in Fig. 27. Microchannel substrate 15A may be produced by pouring a PDMS solution to mold 903 and heating and curing the PDMS solution. In this example, microchannel substrate 15A includes a non-branched channel including one inlet and one outlet. However, the microchannel substrate may include a plurality of inlets and/or a plurality of outlets and may be of a branched type.

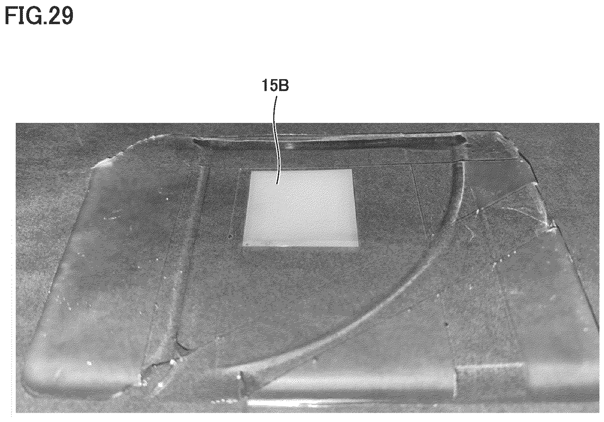

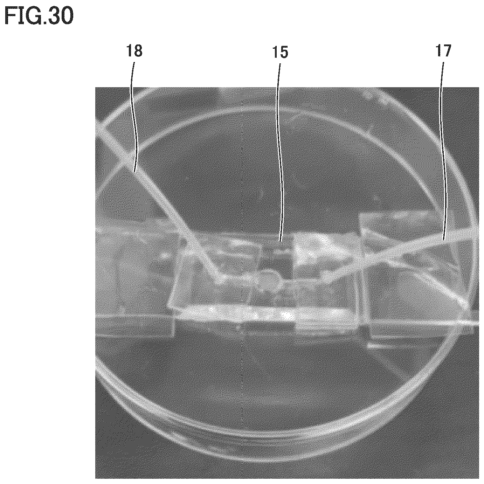

Fig. 29 is a diagram illustrating an example of an accumulation substrate. An accumulation substrate 15B as illustrated in Fig. 29 is joined to microchannel substrate 15A. Accumulation substrate 15B is the same as accumulation substrate 13 (see Figs. 8 and 9) according to the embodiment. Fig. 30 is an image of accumulation kit 15 produced in practice.

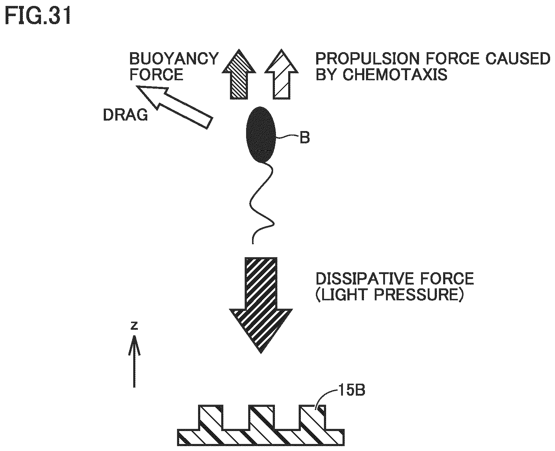

Fig. 31 is a diagram for explaining an accumulation mechanism of a plurality of types of microorganisms according to the modification. In a case where the microorganisms are bacteria that have chemotaxis, a drag caused by a fluid (pressure drive flow) acts on the bacteria in addition to the buoyancy force, the force caused by the Brownian motion, and the propulsion force of the bacteria themselves acting thereon.

In the present modification, the light pressure acting downward on the bacteria through the irradiation with the laser light is set to be greater than an upward component of the drag. In other words, the downward component of the light pressure is greater than (the upward component of) the buoyancy force, is greater than the upward component of the force caused by the Brownian motion, is greater than the upward component of the propulsion force caused by the chemotaxis, and is greater than the upward component of the drag. More preferably, the downward component of the light pressure is greater than the sum of the upward component of the buoyancy force, the upward component of the force caused by the Brownian motion, the upward component of the propulsion force caused by the chemotaxis, and the upward component of the drag. It is possible to realize accumulation of a plurality of bacteria including bacteria that have chemotaxis even in a fluid by setting the light pressure to be a sufficiently large value in this manner.

Additionally, active bacteria (for example, bacteria with active cell division) are constantly supplied to accumulation kit 15 by using the microchannel. According to the present modification, it is possible to accumulate active bacteria with high efficiency even from a sample at a low concentration. Also, it is possible to achieve a high survival rate as in the embodiment.

### [Clauses]

Finally, various aspects of the present disclosure will be collectively described as supplements.

### (Clause 1)

A microorganism accumulation method of accumulating a plurality of types of microorganisms included in a liquid sample, the method including:
preparing a substrate provided with a plurality of pores, each of the plurality of pores having an opening and a depth, the opening being capable of capturing at least one microorganism for each of the plurality of types, the depth extending in a direction including a vertically downward component;
introducing the liquid sample onto the substrate;
setting an irradiation condition for non-resonant light that is light outside an electronic resonance wavelength range of the plurality of types of microorganisms; and
irradiating the plurality of pores with the non-resonant light through the liquid sample in accordance with the irradiation condition,
in which a region irradiated with the non-resonant light in the plurality of pores does not include a photothermal conversion material that converts the non-resonant light into heat, and
the setting includes setting, for the plurality of types of microorganisms, intensity of the non-resonant light within an irradiation range of the non-resonant light such that a magnitude of a vertically downward component of a light-induced force caused by irradiation with the non-resonant light (i) is greater than a magnitude of a vertically upward component of a buoyancy force caused by the liquid sample and (ii) is greater than a magnitude of a vertically upward component of a force caused by a Brownian motion of molecules in the liquid sample.

### (Clause 2)

The microorganism accumulation method according to Clause 1,
in which the setting includes setting, for the plurality of types of microorganisms, the intensity within the irradiation range such that the magnitude of the vertically downward component of the light-induced force is greater than a sum of (i) the magnitude of the vertically upward component of the buoyancy force and (ii) the magnitude of the vertically upward component of the force caused by the Brownian motion.

### (Clause 3)

The microorganism accumulation method according to Clause 1 or 2,
in which the plurality of types of microorganisms include microorganisms that have chemotaxis, and
the setting includes setting, for the microorganisms that have chemotaxis, the intensity within the irradiation range such that the magnitude of the vertically downward component of the light-induced force is yet greater than a magnitude of a vertically upward component of a propulsion force caused by the chemotaxis.

### (Clause 4)

The microorganism accumulation method according to Clause 3,
in which the setting includes setting, for the microorganisms that have chemotaxis, the intensity within the irradiation range such that the magnitude of the vertically downward component of the light-induced force is greater than a sum of (i) the magnitude of the vertically upward component of the buoyancy force, (ii) the magnitude of the vertically upward component of the force caused by the Brownian motion, and (iii) the magnitude of the vertically upward component of the propulsion force caused by the chemotaxis.

### (Clause 5)

The microorganism accumulation method according to Clause 3 or 4,
in which the depth of each of the plurality of pores is defined such that the microorganism that has the chemotaxis and is captured in each pore does not escape with the propulsion force caused by the chemotaxis.

### (Clause 6)

The microorganism accumulation method according to any one of claims 1 to 5,
in which the introducing is distributing the liquid sample on the substrate, and
the setting includes setting, for the plurality of types of microorganisms, the intensity within the irradiation range such that the magnitude of the vertically downward component of the light-induced force is yet greater than a magnitude of a vertically upward component of a drag caused by the liquid sample.

### (Clause 7)

The microorganism accumulation method according to claim 6,
in which the setting of includes setting, for the plurality of types of microorganisms, the intensity within the irradiation range such that the vertically downward component of the light-induced force is greater than a sum of (i) the magnitude of the vertically upward component of the buoyancy force, (ii) the magnitude of the vertically upward component of the force caused by the Brownian motion, and (iii) the magnitude of the vertically upward component of the drag of the liquid sample.

### (Clause 8)

The microorganism accumulation method according to any one of Clauses 1 to 7,
in which the setting further includes setting the irradiation range to include entire openings of two or more pores among the plurality of pores.

### (Clause 9)

The microorganism accumulation method according to any one of Clauses 1 to 8,
in which adjacent pores among the plurality of pores do not communicate with each other.

### (Clause 10)

The microorganism accumulation method according to any one of Clauses 9,
in which the introducing of the liquid sample includes forming a closed system in which the liquid sample and gas around the liquid sample are not in contact with each other.

### (Clause 11)

The microorganism accumulation method according to any one of Clauses 1 to 10,
in which the introducing includes preparing a concentration of the plurality of types of microorganisms in the liquid sample to such a high concentration that the plurality of types of microorganisms are accumulated in a range wider than the irradiation range.

### (Clause 12)

A microorganism accumulation system that accumulates a plurality of types of microorganisms included in a liquid sample, the microorganism accumulation system including:
a substrate that is provided with a plurality of pores, each of the plurality of pores having an opening and a depth, the opening being capable of capturing at least one microorganism for each of the plurality of types, the depth extending in a direction including a vertically downward component;
a light source that irradiates the plurality of pores with non-resonant light through the liquid sample in a state where the liquid sample is disposed on the substrate, the non-resonant light being light outside an electronic resonance wavelength range of the plurality of types of microorganisms; and
a controller that controls the light source,
in which a region irradiated with the non-resonant light in the plurality of pores does not include a photothermal conversion material that converts the non-resonant light into heat, and
the controller sets, for the plurality of types of microorganisms, intensity of the non-resonant light within an irradiation range of the non-resonant light such that a magnitude of a vertically downward component of a light-induced force caused by irradiation with the non-resonant light (i) is greater than a magnitude of a vertically upward component of a buoyancy force caused by the liquid sample and (ii) is greater than a magnitude of a vertically upward component of a force caused by a Brownian motion of molecules in the liquid sample.

The embodiment disclosed herein is an illustrative example and should be considered as a non-restrictive example in any sense. The scope of the present disclosure is indicated by the claims rather than the above description of the embodiment and is intended to include all modifications within the meanings and the scope equivalent to the claims.

### REFERENCE SIGNS LIST

1 Accumulation kit; 11 Base substrate; 12 Holding frame; 121 Through-hole; 13 Accumulation substrate; 131 Pore; 14 Cover glass; 15 Accumulation kit; 15A Microchannel substrate; 15B Accumulation substrate; 16 Pump; 17, 18 Capillary; 2 XYZ stage; 3 Adjustment mechanism; 4 Laser light source; 5 Dichroic mirror; 6 Objective lens; 7 Illumination light source; 8 Camera; 9 HMI; 10 Controller; 101 Processor; 102 Memory; 103 Input/output port; 100, 200 Accumulation system; 901 Mold; 902 Mixed solution; 903 Mold.

## Claims

1. A microorganism accumulation method of accumulating a plurality of types of microorganisms included in a liquid sample, the method comprising:
preparing a substrate provided with a plurality of pores, each of the plurality of pores having an opening and a depth, the opening being capable of capturing at least one microorganism for each of the plurality of types, the depth extending in a direction including a vertically downward component;
introducing the liquid sample onto the substrate;
setting an irradiation condition for non-resonant light that is light outside an electronic resonance wavelength range of the plurality of types of microorganisms; and
irradiating the plurality of pores with the non-resonant light through the liquid sample in accordance with the irradiation condition, wherein
a region irradiated with the non-resonant light in the plurality of pores does not include a photothermal conversion material that converts the non-resonant light into heat, and
the setting includes setting, for the plurality of types of microorganisms, intensity of the non-resonant light within an irradiation range of the non-resonant light such that a magnitude of a vertically downward component of a light-induced force caused by irradiation with the non-resonant light (i) is greater than a magnitude of a vertically upward component of a buoyancy force caused by the liquid sample and (ii) is greater than a magnitude of a vertically upward component of a force caused by a Brownian motion of molecules in the liquid sample.

2. The microorganism accumulation method according to claim 1, wherein
the setting includes setting, for the plurality of types of microorganisms, the intensity within the irradiation range such that the magnitude of the vertically downward component of the light-induced force is greater than a sum of (i) the magnitude of the vertically upward component of the buoyancy force and (ii) the magnitude of the vertically upward component of the force caused by the Brownian motion.

3. The microorganism accumulation method according to claim 1, wherein
the plurality of types of microorganisms include microorganisms that have chemotaxis, and
the setting includes setting, for the microorganisms that have chemotaxis, the intensity within the irradiation range such that the magnitude of the vertically downward component of the light-induced force is yet greater than a magnitude of a vertically upward component of a propulsion force caused by the chemotaxis.

4. The microorganism accumulation method according to claim 3, wherein
the setting includes setting, for the microorganisms that have chemotaxis, the intensity within the irradiation range such that the magnitude of the vertically downward component of the light-induced force is greater than a sum of (i) the magnitude of the vertically upward component of the buoyancy force, (ii) the magnitude of the vertically upward component of the force caused by the Brownian motion, and (iii) the magnitude of the vertically upward component of the propulsion force caused by the chemotaxis.

5. The microorganism accumulation method according to claim 3, wherein
the depth of each of the plurality of pores is defined such that the microorganism that has the chemotaxis and is captured in each pore does not escape with the propulsion force caused by the chemotaxis.

6. The microorganism accumulation method according to claim 1, wherein
the introducing is distributing the liquid sample on the substrate, and
the setting includes setting, for the plurality of types of microorganisms, the intensity within the irradiation range such that the magnitude of the vertically downward component of the light-induced force is yet greater than a magnitude of a vertically upward component of a drag caused by the liquid sample.

7. The microorganism accumulation method according to claim 6, wherein
the setting includes setting, for the plurality of types of microorganisms, the intensity within the irradiation range such that the magnitude of the vertically downward component of the light-induced force is greater than a sum of (i) the magnitude of the vertically upward component of the buoyancy force, (ii) the magnitude of the vertically upward component of the force caused by the Brownian motion, and (iii) the magnitude of the vertically upward component of the drag of the liquid sample.

8. The microorganism accumulation method according to any one of claims 1 to 7, wherein
the setting further includes setting the irradiation range to include entire openings of two or more pores among the plurality of pores.

9. The microorganism accumulation method according to any one of claims 1 to 7, wherein
adjacent pores among the plurality of pores do not communicate with each other.

10. The microorganism accumulation method according to any one of claims 1 to 7, wherein
the introducing includes forming a closed system in which the liquid sample and gas around the liquid sample are not in contact with each other.

11. The microorganism accumulation method according to any one of claims 1 to 7, wherein
the introducing of the liquid sample includes preparing a concentration of the plurality of types of microorganisms in the liquid sample to such a high concentration that the plurality of types of microorganisms are accumulated in a range wider than the irradiation range.

12. A microorganism accumulation system that accumulates a plurality of types of microorganisms included in a liquid sample, the microorganism accumulation system comprising:
a substrate that is provided with a plurality of pores, each of the plurality of pores having an opening and a depth, the opening being capable of capturing at least one microorganism for each of the plurality of types, the depth extending in a direction including a vertically downward component;
a light source that emits non-resonant light to the plurality of pores through the liquid sample in a state where the liquid sample is disposed on the substrate, the non-resonant light being light outside an electronic resonance wavelength range of the plurality of types of microorganisms; and
a controller that controls the light source, wherein
a region irradiated with the non-resonant light in the plurality of pores does not include a photothermal conversion material that converts the non-resonant light into heat, and
the controller sets, for the plurality of types of microorganisms, intensity of the non-resonant light within an irradiation range of the non-resonant light such that a magnitude of a vertically downward component of a light-induced force caused by irradiation with the non-resonant light (i) is greater than a magnitude of a vertically upward component of a buoyancy force caused by the liquid sample and (ii) is greater than a magnitude of a vertically upward component of a force caused by a Brownian motion of molecules in the liquid sample.
